# FASCICULE DE BREVET EUROPEEN

(11) **EP 0 806 484 B1**
(45) Date de publication et mention de la délivrance du brevet: **12.04.2006**
(21) Numéro de dépôt: 97110543.2
(22) Date de dépôt: 05.06.1990
(51) Int. Cl.: C12Q 1/70, C12Q 1/68, A61K 39/21, C07K 16/10, G01N 33/577, G01N 33/569, A61K 31/70, A61K 39/42, C12N 15/49

(54) **Synthèse de protéines ou de polypeptides codés par une séquence nucléotidique de HIV-1, HIV-2 ou SIV.**
Protein oder Polypeptidsynthese kodiert durch eine Nukleinsaüresequenz von HIV-1, HIV-2 oder SIV.
Synthesis of proteins or polypeptides encoded by a sequence of HIV-1, HIV-2 or SIV.

(30) Priorité: 02.06.1989 FR 8907354; 20.09.1989 FR 8912371
(43) Date de publication de la demande: 12.11.1997
(62) Demande divisionnaire de: 90401520.3
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Moncany, Maurice, 75019 Paris (FR); Montagnier, Luc, 92350 Le Plessis-Robinson (FR)
(74) Mandataire: Desaix, Anne

(56) Documents cités:
- EP-A- 0 229 701
- EP-A- 0 269 445
- EP-A- 0 269 520
- EP-A- 0 272 098
- WO-A-88/05440
- WO-A1-86/02383
- OU C-H ET AL: "DNA amplification for direct detection of HIV-1 in DNA of peripheral blood mononuclear cells" SCIENCE, vol. 239, 15 janvier 1988 (1988-01-15), pages 295-297, XP002135449
- RAYFIELD M ET AL: "Mixed human immunodeficiency virus (HIV) infection in an individual: Demonstration of both HIV type 1 and 2 proviral sequences by using polymerase chain reaction" THE JOURNAL OF INFECTIOUS MEDICINE, vol. 158, no. 9, 6 décembre 1988 (1988-12-06), pages 1170-76, XP002135450
- KWOK S ET AL: "IDENTIFICATION OF HUMAN IMMUNODEFICIENCY VIRUS SEQUENCES BY USING IN VITRO ENZYMATIC AMPLIFICATION AND OLIGOMER CLEAVAGE DETECTION" JOURNAL OF VIROLOGY,US,NEW YORK, US, vol. 61, no. 5, 1 mai 1987 (1987-05-01), pages 1690-1694, XP000616284 ISSN: 0022-538X
- MYERS ET AL: 'Human retroviruses and AIDS 1988', 1988, LOS ALAMOS LABORATORY, NEW MEXICO, USA * page III.31 *
- FIELDS ET AL: 'VIROLOGY (THIRD EDITION)', LIPPINCOTT-RAVEN, PHILADELPHIA, NEW-YORK, USA * page 1884 - page 1885 *

## Description

La présente invention est relative à des séquences oligonucléotidiques utilisables pour la mise en oeuvre de techniques d'amplification de séquences nucléiques spécifiques de rétrovirus d'immunodéficience humaine du type HIV ou de rétrovirus d'immunodéficience du singe du type SIV.

L'isolement et la caractérisation de rétrovirus regroupés sous les désignations HIV-1 et HIV-2 ont été décrits dans les demandes de brevet, européen n° 85/905.513.9 (HIV-1) et n° 87/400.151.4 et EP 0269520 (HIV-2). Ces rétrovirus ont été isolés chez plusieurs malades présentant des symptômes d'une lymphadénopathie ou d'un Syndrome d'Immunodeficience Acquise (SIDA).

Les rétrovirus du type HIV-2 comme les rétrovirus du type HIV-1, se caractérisent par un tropisme pour les lymphocytes T4 humains et par un effet cytopathogène à l'égard de ces lymphocytes lorsqu'ils s'y multiplient, pour alors causer entre autres des polyadénopathies généralisées et persistantes, ou un SIDA.

Un autre rétrovirus, dénommé SIV-1, cette dénomination remplaçant la dénomination antérieurement connue STLV-III, a été isolé chez le singe macaque rhésus (M.D. DANIEL et al. Science, 228, 1201 (1985) ; N.L. LETWIN et al, Science, 230, 71 (1985) sous l'appellation "STLV-IIImac").

Un autre rétrovirus, désigné "STLV-III_{AGM}", (ou SIV_{AGM}) a été isolé chez des singes verts sauvages. Mais contrairement aux virus présents chez le singe macaque rhésus, la présence de STLV-III_{AGM} ne semble pas induire une maladie du type SIDA chez le singe vert d'Afrique.

Pour la commodité du langage, ces virus ne seront plus désignés dans ce qui suit que par l'expression SIV (l'expression SIV est l'abréviation anglaise de "Simian Immunodeficency Virus" (Virus d'immunodéficience du singe) éventuellement suivie d'une abréviation désignant l'espèce de singe dont ils sont issus par exemple "MAC" pour le macaque" ou "AGM" pour le singe vert d'Afrique (abréviation de "African Green Monkey").

Une souche du rétrovirus SIV-1Mac à été déposée à la C.N.C.M le 7 février 1986 sous le n° I-521.

La poursuite de l'étude des rétrovirus HIV-1 et HIV-2 a également conduit à l'obtention de séquences d'ADN complémentaires (ADNc) des ARN de leur génome. La séquence nucléotidique complète d'un ADNc d'un rétrovirus représentatif de la classe HIV-2 (HIV-2 ROD) a été déposée le 21/02/1986 à la C.N.C.M. sous le n° 1-522, sous le nom de référence LAV-2 ROD.

De même, la séquence nucléotidique complète d'un ADNc d'un rétrovirus représentatif de la classe HIV-1 est décrite par WAIN HOBSON, SONIGO, COLE, DANOS et ALIZON dans CEll (janvier 1985).

Egalement pour la commodité du langage, les virus du type HIV-1 et HIV-2 seront parfois désignés dans ce qui suit par l'expression HIV.

Les méthodes de diagnostic in vitro des infections par des virus du type HIV-1 ou HIV-2 existant actuellement, font appel à la détection d'anticorps ANTI-HIV-1 ou anti-HIV-2 éventuellement présents dans un prélèvement biologique (biopsie) ou dans un fluide biologique, par exemple dans un sérum obtenu, à partir du patient à l'étude, par mise en contact de ce fluide biologique avec des extraits ou antigènes d'HIV-1 ou d'HIV-2, dans des conditions permettant la production d'une réaction immunologique éventuelle de ces extraits ou antigènes avec ces anticorps.

De telles méthodes de diagnostic risquent d'être faussement négatives, en particulier dans le cas d'une infection récente d'un individu par les virus du type HIV.

Les techniques d'amplification génique sont d'un appoint considérable pour la mise au point de méthodes de diagnostic in vitro particulièrement sensibles de maladies virales. Parmi ces techniques d'amplification génique, on peut citer la technique PCR (Polymérase Chain Reaction) telle que décrite dans les demandes de brevet européen n° 86/302.298.4 du 27/03/1986 et n° 87/300.203.4 du 09/01/1987, ou encore la technique dite "Qβreplicase" décrite dans Biotechnology, vol.6, page 1197 (octobre 1988) et celle procédant à l'aide d'une ARN polymérase (T7RNA polymérase) décrite dans la demande de brevet international n° WO89/01050. Ces techniques permettent d'améliorer la sensibilité de détection des acides nucléiques des virus, et nécessitent l'utilisation d'amorces de synthèse spécifiques.

Pour la recherche des virus du type HIV, le choix des amorces est problématique. En effet, du fait de la grande variabilité des séquences de nucléotides du génome viral, une amorce conforme à la séquence connue d'un isolat donné d'un virus du type HIV peut faillir à l'amplification de certains variants viraux du type HIV. D'autre part, même si une amorce est choisie dans une région conservée du génome d'un virus HIV à un autre, son "bon fonctionnement" n'est pas pour autant assuré et peut donner lieu à de mauvais rendements d'amplification.

La présente invention fournit précisément des amorces oligonucléotidiques permettant, entre autres, l'amplification du génome de tous virus du type HIV et SIV, avec des rendements considérés comme maximum dans l'état actuel de la technique et surtout évitant la présence de nombreuses bandes aspécifiques.

Les amorces utilisées dans la présente invention sont à la fois spécifiques des virus du groupe HIV-1 et/ou des virus des groupes HIV-2 et SIV, et sont insensibles aux variations du génome de ces virus.

La présente invention a pour objet l'utilisation des amorces oligonucléotidiques, d'environ 15 à 30 nucléotides, utilisables pour l'amplification génomique des virus du type HIV-1 et/ou du type HIV-2 et SIV.

L'invention met en oeuvre toute séquence nucléotidique caractérisée en ce que sa séquence :
- soit est choisie parmi celles qui sont contenues dans l'une des séquences nucléotidiques comprises dans le gène gag des virus HIV-1 Bru, HIV-1 Mal, HIV-1 Eli, HIV-2 ROD et SIV MAC, et plus particulièrement parmi celles qui sont contenues dans les enchaînements nucléotidiques définis ci-après,
- soit (notamment pour les séquences les plus longues) contient l'une des séquences nucléotidiques susdites issues de HIV-1 Bru, ou HIV-1 Mal, ou HIV-1 Eli ou HIV-2 ROD ou SIVMac , ou contient une séquence nucléotidique complémentaire de l'une de ces dernières séquences, étant entendu que les nucléotides supplémentaires éventuels qui "débordent" la séquence nucléotidique du genre en question, du côté des extrémités 3' ou 5', coïncident de préférence avec ceux qui se trouvent placés en deçà des extrémités 5'
ou 3' correspondant au sein même de la séquence complète des virus du type HIV-1, HIV-2 ou de SIV MAC, sus-mentionnés,
- soit, si cette séquence nucléotidique n'est pas identique à l'une des séquences nucléotidiques susdites, ou n'est pas complémentaire de l'une de ces séquences, est néanmoins susceptible de s'hybrider avec une séquence nucléotidique issue des virus HIV-1 Bru,HIV-1 Mal, HIV-1 Eli, et/ou avec une séquence-nucléotidique issue du virus HIV-2 ROD ou SIV MAC sus-mentionnée. L'hybridation peut s'effectuer à une température de 60°C ± 1°C (de préférence 60°C ± 0,5°C), préconisée pour un optimum de rendement.

La numérotation des nucléotides mentionnés ci-dessous correspond à celle utilisée dans le manuel de référence "Human Retrovirus and AIDS-1989" édité par le "Los Alamos National Laboratory- New Mexico - USA".

Les séquences des virus HIV-1 Mal, HIV-1 Eli ont été décrites par MONTAGNIER, SONIGO, WAIN-HOBSON et ALIZON dans la demande de brevet européen n° EP 0 253 701.

Les séquences de l'invention sont synthétisées sur synthétiseur commercialisé par Applied Biosystems (méthode phosphoro-amidites, ou sur tout autre appareil utilisant une méthode semblable.

L'invention concerne plus particulièrement l'utilisation des séquences oligonucléotidiques caractérisées par les enchaînements nucléotidiques suivants (représentés dans le sens 5' → 3'; les initiales "S" et "AS" indiquent si l'oligonucléotide est sens ou antisens, c'est-à-dire si l'oligonucléotide est orienté respectivement dans le sens 5'o→ 3' ou dans le sens 3' o→ 5') :
1°) séquences communes aux génomes.des virus HIV-1, HIV-2 et SIV (les séries de chiffres espacées d'un trait indiquent la position des nucléotides sur les génomes correspondant respectivement aux virus HIV-1 Bru, HIV-1 Mal, HIV-1 Eli, HIV-2 ROD et SIV) :
   - séquences spécifiques du gène gag du génome des virus sus-mentionnés (gène codant pour un groupe d'antigènes spécifiques du nucléoide de ces virus).

Certaines variantes peuvent être apportées sur certaines positions des séquences nucléotidiques indiquées ci-dessous, sans que les propriétés d'hybridation de ces séquences nucléotidiques avec les gènes des virus du type HIV et/ou SIV soient affectées. Les séquences nucléotidiques comportant ces variantes sont représentées en-dessous des séquences nucléotidiques initiales dont elles dérivent par remplacement d'une ou plusieurs bases. Les bases modifiées par rapport à celles des séquences nucléotidiques initiales sont indiquées en toute lettre à la verticale des positions correspondant aux bases qui ont été remplacées dans ces séquences initiales ; tandis que les bases des séquences initiales qui n'ont pas été remplacées dans les séquences comportant ces variantes sont indiquées à l'aide de pointillés.

La synthèse des amorces se fait en utilisant toutes les variantes simultanément. C'est le mélange de toutes les variantes pour une séquence donnée qui est utilisé dans les tests.

MMy1 :   TGG CGC CCG AAC AGG GAC
         ... ... .T. ... ... ...
         S, 636-653, 635-652, 636-653, 859-876, 834-851

MMy2 :    GGC CAG GGG GAA AGA AAA A
          ... .C. .C. ... ... ... .
          ... ... .A. ... ... ... .
          S,854-872,864-888,848-872,1160-1184, 1124-1148

MMy3 :    TGC CCA TAC AAA ATG TTT TA
          ... ... C.. T.T ... ... ..
     AS,900-881,916-897,900-881,1212-1193,1176-1157

MMy4 : TGC ATG GCT GCT TGA TG
... ..A ... ..C ..G ..
AS,1385-1369,1419-1403,1385-1369,1703-1687,1667-1651

MMy4B : CTT TGC ATG GCT GCT TGA TG
..C ... ..A ... ..C ..G ..
AS, 1388-1369, 1421-1403, 1388-1369, 1706-1687,
1670-1651,

MMy4Bbis : CAT CAA GCA GCC ATG CAA AG
..C ..G ... ..T ... ..G ..
S, 1369-1388, 1403-1421, 1369-1388,
1687-1706, 1651-1670,

MMy28 : AGG GCT GTT GGA AAT GTG G
... ... ... ... ..G ... .
S, 2021-2039, 2055-2073, 2024-2042, 2329-2349,
2299-2318,

MMy28bis : CCA CAT TTC CAG CAT CCC T
... ... ... ... ..G ... .
... ... ... ... ..C ... .
AS, 2039-2021, 2073-2055, 2042-2024, 2349-2329,
2318-2299

L'invention a également pour objet l'utilisation dans le procédé de synthèse des séquences (ou amorces) possédant une structure nucléotidique complémentaire de celles des amorces définies ci-dessus.

Elle concerne également l'utilisation dans le procédé de synthèse des séquences nucléotidiques présentant certaines mutations par rapport à celles définies ci-dessus sans que les propriétés d'hybridation, telles que définies ci-dessus, de ces séquences soient modifiées. Le pourcentage de nucléotides différents de ceux constituant les séquences décrites ci-dessus, sans pour autant affecter les propriétés d'hybridation des séquences de l'invention, peut atteindre 40 %.

D'une manière générale, dans le cas d'une amorce (primer) sens (S), un plus grand nombre de mutations seront tolérées du côté 5' que du côté 3' de l'amorce, le côté 3' devant s'hybrider parfaitement avec un brin déterminé d'une séquence nucléique pour permettre l'amplification de cette séquence. Dans le cas d'une amorce anti-sens(AS), la tolérance est permise du côté 3'.

Les amorces telles que définies ci-dessus et peuvent comporter une conservation d'au moins 5 bases de chaque côté, la partie médiane comportant des modifications, sans que les propriétés d'hybridation ci-dessus soient modifiées.

Une des caractéristiques des amorces oligonucléotidiques utilisées dans l'invention est de donner une bande d'amplification nette, dépourvue généralement de bandes aspécifiques lorsque les indications techniques d'utilisation décrites dans la présente invention sont mises en oeuvre. Ce fait est dû à la longueur des amorces pouvant atteindre 27 bases ce qui accroît la spécificité d'hybridation, ainsi qu'aux conditions d'utilisation drastiques qui permettent d'éliminer les associations parasites. La spécificité pour chaque type de virus est fonction, outre du pourcentage d'homologie avec la matrice de référence, de la longueur des amorces, qui peuvent atteindre, pour un rendement acceptable, jusqu'à 40 bases.

L'invention s'étend également à l'utilisation des amorces telles que décrites ci-dessus liées au niveau de leur extrémité 5' à un promoteur pour la mise en oeuvre d'une méthode d'amplification génomique par synthèse de multiple copies d'ADN ou d'ARN telle que décrite dans la demande de brevet européenne n° 88/307.102.9 du 01/08/1988.

L'invention a notamment pour objet l'utilisation des amorces décrites ci-dessus pour la mise en oeuvre d'un procédé d'amplification génique de séquences nucléiques de virus du type HIV-1 et/ou HIV-2, et/ou Le procédé d'amplification génique comprend principalement les étapes suivantes :
- une étape d'extraction de l'acide nucléique à détecter appartenant au génome du virus du type HIV-1, HIV-2 ou SIV et, le cas échéant, une étape de traitement à l'aide d'une transcriptase inverse dudit acide nucléique si ce dernier est sous forme d'ARN afin d'obtenir un acide nucléique double brin (cette dernière étape étant encore désignée ci-dessous par étape de rétro-transcription de l'ARN viral),
- un cycle comprenant les étapes suivantes :
   - dénaturation de l'acide nucléique double brin à détecter , ce qui conduit à la formation d'un acide nucléique simple brin,
   - hybridation de chacun des brins d'acide nucléique, obtenus lors de l'étape de dénaturation précédente, avec au moins une amorce selon l'invention, par mise en contact des brins sus-mentionnés avec au moins un couple d'amorces selon l'invention dans les conditions d'hybridation définies ci-dessous,
   - formation à partir des amorces des ADN complémentaires aux brins sur lesquels elles sont hybridées en présence d'un agent de polymérisation (ADN polymérase) et de quatre nucléosides triphosphate (dNTP) différents, ce qui conduit à la formation d'un plus grand nombre d'acides nucléiques double brin à détecter qu'à l'étape de dénaturation précédente, ce cycle étant répété un nombre de fois déterminé pour obtenir ladite séquence nucléique à détecter éventuellement présente dans l'échantillon biologique dans une proportion suffisante pour permettre sa détection,

L'étape d'hybridation décrite ci-dessus est avantageusement réalisée à 60°C pendant 1 minute 30 secondes dans le tampon "10 X buffer" dont la composition (en concentration finale d'utilisation) est indiquée ci-dessous.

Les génomes des virus HIV et SIV se présentent sous forme d'ARN ou d'ADN en fonction de la localisation du virus dans l'organisme.

Lorsque le virus est situé à l'intérieur des cellules de l'organisme, notamment à l'intérieur des cellules sanguines, son ARN est recopié en ADN par une transcriptase inverse. En revanche, le génome des virus du type HIV en milieu extracellulaire, notamment dans le sang, demeure sous forme d'ARN.

L'étape d'extraction de l'ADN viral contenu dans les cellules de l'échantillon biologique préconisée par les inventeurs - outre la méthode classique au phénol chloroforme - présente les étapes suivantes :
- suspension du culot cellulaire dans 0,5 ml d'eau pyrolisée dans un Potter gros piston,
- broyage des cellules dit par "aller et retour",
- adjonction Triton X100 pour 1 concentration finale de 0,1 %,
- dénaturation à la chaleur durant 15 à 25 minutes à 100°C,
- centrifugation courte pour n'éliminer que les débris cellulaires,
- précipitation de l'ADN durant la nuit à -20°C par adjonction de 2,5 volumes d'éthanol absolu et de 10 % du volume final d'acétate de sodium 3 Molaires. L'ADN est ensuite récupéré puis resuspendu dans l'eau pyrolysée après avoir été lavé 2 fois par de l'éthanol à 70°. Il est à noter que cette méthode permet la précipitation conjointe des ADN et des ARN, ce qui autorise la détection du message génomique des virus de types HIV ou SIV par utilisation de la méthode dite "PCR directe ADN" ou par celle dite de "PCR-ARN".

L'étape d'extraction de l'ARN viral est généralement effectuée de manière classique connue de l'homme de l'Art.

Après extraction de l'ARN, une étape supplémentaire de transformation de l'ARN, monobrin en ADN double brin est nécessaire à effectuer lorsque le diagnostic in vitro de l'invention est réalisé à partir d'échantillons biologiques contenant les virus du type HIV-1 et/ou HIV-2 et/ou SIV dont les génomes sont sous forme d'ARN.

Cette transformation de l'ARN en ADN est réalisée par traitement de l'ARN obtenu après extraction de l'échantillon biologique, notamment du sérum, dans un milieu approprié à l'aide d'une transcriptase inverse.

L'étape de rétro-transcription de l'ARN viral est réalisée de la manière suivante :
- 10 *µ*g d'ARN extrait resuspendu dans de l'eau est mis en présence du couple d'amorces à la concentration de 40 *µ*M chacun, dans un volume final de 40 *µ*l. L'ensemble est dénaturé à 100°C durant 10 minutes puis plongé dans de l'eau glacée,
- l'on rajoute 10 *µ*l du mélange suivant : 5 *µ*l du tampon "10 X buffer" décrit ci-dessous + 1 unité de reverse-transcriptase (d'AMV (Avian Myeloblastosis Virus) ou de MuMLV (Moloney Leukemia Virus)) + 1 unité de Taq-polymérase + 1 *µ*l du mélange des 4 dNTP à 25 mM chacun + de l'eau Q.S.P. 10 *µ*l. Le volume final est donc de 50 *µ*l.

Cette réaction s'effectue en deux étapes :
- a) 1ère étape : fabrication de l'ADNc par action de la réverse transcriptase à 42°C durant 13 minutes,
- b) 2ème étape : amplification génique classique: on chauffe à 95°C durant 3 minutes pour détruire la réverse-transcriptase et permettre l'étape de déshybridation/hybridation, puis on démarre le cycle décrit précédemment pour l'amplification génique.

L'étape de dénaturation est réalisée en présence du (ou des) couple(s) d'amorces (ou de primers) de l'invention. En effet, comme il a été précisé ci-dessus, une des caractéristiques des oligonucléotides (ou primers), de l'invention est de donner une bande d'amplification nette, dépourvue généralement de bandes aspécifiques, lorsqu'ils sont utilisés dans les conditions qui suivent :
- hybridation : les primers (1*µ*l d'une solution à 40 *µ*molaire (40 *µ*M) de chaque primer) sont mis en présence de l'ADN-matrice (100 à 300 ng) pour la première étape de dénaturation-réassociation ; on chauffe, durant 10 minutes à 100°C puis on plonge les tubes contenant ce mélange d'ADN-matrice et de primers dans de l'eau contenant de la glace afin d'augmenter le taux de réassociation ADN-matrice/primers. Les primers doivent être utilisés à une concentration finale dans l'étape d'amplification qui suit de 0,8 *µ*M chaque.
- amplification : on ajoute au milieu précédent les 4 A dNTP chacun étant utilisé à 0,5 *µ*molaire en solution finale (50 *µ*l), et une unité de Taq-polymérase pour un milieu réactionnel de 50 *µ*l ;cette étape est réalisée dans un tampon d'amplification de la présente invention, généralement désigné sous le nom de "10 X buffer" dont la composition (lorsqu'il est dilué au 1/10°) est la suivante : Tris-HCl, pH 8,9 : 50 mM ; (NH₄)₂ SO₄ : 15 mM ; MgCl2 : 5 mM ; *β*-mercapto-éthanol : 10 mM ; gélatine : 0,25 mg/ml. On additionne 5 *µ*l de ce tampon et de l'eau q.s.p. 50 *µ*l au milieu précédent.

Les cycles d'amplification sont réalisés de la manière suivante : 30 à 40 cycles composés de :
- 94°C durant 10 secondes (dénaturation),
- 60°C durant 1 minute 30 (hybridation),
- 78°C durant 1 minute 30 (élongation).

Le tout sera suivi par un cycle unique à 78°C durant 15 minutes.

La précision des températures indiquées à ± 0,3°C près, ainsi que leur stabilité durant les différents cycles, représentent des conditions essentielles pour l'obtention des rendements maximum ainsi que l'absence de bandes aspécifiques.

La concentration optimale d'ADN est de 100 à 300 ng pour de l'ADN génomique extrait de cellules (de patients ou en culture, de mammifères ou autres).

Il va de soi que les conditions qui précèdent représentent des conditions optimales pour un milieu réactionnel final de 50 *µ*l, et que ces conditions peuvent être modifiées en fonction du volume final du milieu réactionnel.

L'agent de polymérisation utilisé dans l'étape d'élongation du cycle est une ADN polymérase thermostable, notamment la Taq polymérase, l'amplifiose de la firme Appligène ou toute ADN-polymérase thermostable pouvant être commercialisée.

D'une manière générale, le cycle de la méthode d'amplification génique de l'invention est répété entre 30 et 40 fois.

Les couples d'amorces , utilisables, à titre d'exemples, pour la méthode d'amplification génique sont les suivants :
- MMy1-MMy4, MMy2-MMy4, MMy1-MMy3, MMy4bis-MMy28bis pour le gène gag,

Toutefois, les combinaisons entre primers "S" et "AS" décrites ci-dessus ne sont par limitatives et peuvent être variées selon le désir de l'utilisateur.

Les tailles des fragments nucléotidiques synthétisés à l'aide des couples d'amorces sus-mentionnés à titre d'exemples, sont indiquées sur le tableau suivant :
(les chiffres indiqués dans le tableau ci-dessous représentent le nombre de nucléotides des fragments synthétisés, et les "tirets" indiquent que les couples d'amorces testés ne permettent pas de caractériser les souches virales correspondantes).

**Tableau I**

| gag | | | gag | |
|---|---|---|---|---|
| :MMy1-MMy3:MMy1-MMy4:MMy2-MMy4:MMy4bis-MMy28bis: | | | | |
| HIV1-BRU: | 265 | 750 | 532 | 671 |
| HIV1-MAL: | 282 | 785 | 556 | 671 |
| HIV1-ELI: | 265 | 750 | 538 | 674 |
| HIV2-ROD: | 354 | 845 | 544 | 663 |
| SIV : | 343 | 844 | 544 | 668 |

L'invention a également pour objet la mise en oeuvre des oligonucléotides tels que décrits ci-dessus et comportant des sucres en conformation a. De tels oligonucléotides présentent la caractéristique d'inverser le sens de la double hélice formée avec la matrice (brin du génome du virus), cette double hélice passant ainsi de l'état "S" à l'état "AS".

L'invention concerne aussi la mise en oeuvre des oligonucléotides décrits ci-dessus dont certains nucléotides sont méthylés et/ou comportent un ou plusieurs atomes de soufre notamment sur les adénines. De tels oligonucléotides présentent la caractéristique d'augmenter la stabilité de la double hélice, et par conséquent de mieux s'hybrider avec le brin d'ADN à amplifier.

L'invention concerne également la mise en oeuvre des oligonucléotides tels que décrits ci-dessus et se présentant sous la forme dite "de bases modifiées" comportant des nucléotides sur lesquels sont greffés de façon covalente des agents chromophores (molécules aromatiques planes telles que l'acridine orange), notamment selon la méthode décrite dans l'article de C. Hélène paru dans "la Vie des Sciences", compte-rendus, série générale, tome 4, n°1, p. 17-37. De tels oligonucléotides présentent la caractéristique d'être facilement détectables, notamment par fluorescence.

L'invention concerne l'utilisation des amorces de l'invention indiquées ci-dessus pour la mise en oeuvre d'un procédé de synthèse de protéines codées par les séquences nucléotidiques amplifiées à l'aide de ces amorces.

Le procédé de synthèse d'une protéine ou d'un polypeptide codé par une séquence nucléotidique du virus HIV-1, HIV-2 ou SIV selon l'invention est caractérisé en ce qu'il comprend les étapes suivantes :
a. synthèse de la séquence nucléotidique par un procédé d'amplification génique de séquences nucléotidiques de virus de type HIV-1, H IV-2 ou SIV, à l'aide d'au moins deux amorces oligonucléotidiques dont les séquences consistent chacune en
   i. une séquence spécifique du gène gag choisie dans une région conservée entre les génomes des virus HIV-1 Bru, HIV-1 Mal, HIV-1 Eli, HIV-2 Rod ét SIV Mac; capable d'hybrider à une température de 60°C ± 1°C avec les génomes des virus HIV-1 Bru, HIV-1 Mal, HIV-1 Eli, HIV-2 Rod et SIV Mac ou,
   ii. une séquence complémentaire d'une séquence telle que définie en i.; et
b. récupération de la séquence nucléotidique ainsi amplifiée et traduction en protéine.

Un procédé de synthèse particulier est celui dans lequel la séquence des amorces a au moins 60% d'identité avec la séquence du gène gag d'un virus HIV-1 Bru, ou HIV-1 Mal, ou HIV-1 Eli, ou HIV-2 ROD ou SIV Mac.

Un autre procédé de synthèse particulier selon l'invention est caractérisé en ce que les amorces oligonucléotidiques comprennent une conservation d'au moins 5 bases de chaque côté de l'amorce par rapport à la séquence du gène gag d'un virus HIV-1 Bru, HIV-1 Mal, HIV-1 Eli, ou HIV-2 ROD ou SIV Mac. et gardent les propriétés d'hybridation avec les génomes des virus HIV-1 Bru, HIV-1 Mal, HIV-1 Eli, HIV-2 Rod et SIV Mac

Un autre procédé selon l'invention est caractérisé en ce que le procédé d'amplification génique comprend les étapes suivantes :
a. une étape d'extraction de l'acide nucléique appartenant au génome du virus de type HIV-1, HIV-2 ou SIV, et éventuellement une étape de traitement à l'aide d'une transcriptase reverse dudit acide nucléique si ce dernier est sous forme d'ARN.
b. un cycle comprenant les étapes suivantes :
   i. dénaturation de l'acide nucléique double brin à détecter, ce qui conduit à la formation d'un acide nucléique simple brin,
   ii. hybridation de chacun des brins d'acide nucléique, obtenus lors de l'étape de dénaturation précédente, avec au moins une amorce nucléotidique, par mise en contact des brins sus-mentionnés avec au moins un couple d'amorces selon l'une des revendications 1 ou 2,
   iii. formation, à partir des amorces, des ADN complémentaires aux brins sur lesquels lesdites amorces sont hybridées en présence d'une ADN polymérase et de quatre nucléosides triphosphates (dNTP) différents, ce qui conduit à la formation d'un plus grand nombre d'acides nucléiques double brin qu'à l'étape de dénaturation précédente,
ce cycle étant répété un nombre de fois déterminé pour obtenir ladite séquence nucléotidique dans une proportion suffisante pour permettre sa détection.

Un autre procédé selon l'invention est caractérisé en ce que les au moins deux amorces oligonucléotidiques ont des séquences consistant chacune en
i. au moins une séquence choisie dans le groupe des séquences sens suivant : 
5'-TGG CGC CCG AAC AGG GAC-3'
5'-TGG CGC CTG AAC AGG GAC-3'
5'-GGC CAG GGG GAA AGA AAA A-3'
5'-GGC CCG GCG GAA AGA AAA A-3'
5'-GGC CAG GAG GAA AGA AAA A-3'
5'-CAT CAA GCA GCC ATG CAA AG-3'
5'-CAC CAG GCA GCT ATG CAG AG-3'
5'-AGG GCT GTT GGA AAT GTG G-3'
5'-AGG GCT GTT GGA AGT GTG G-3'
 et au moins une séquence choisie dans le groupe des séquences antisens suivant 
3'-TGC CCA TAC AAA ATG TTT TA-5''
3'-TGC CCA CAC TAT ATG TTT TA-5'
3'-TGC ATG GCT GCT TGA TG-5'
3'-TGC ATA GCT GCC TGG TG-5'
3'-CTT TGC ATG GCT GCT TGA TG-5'
3'-CTC TGC ATA GCT GCC TGA TG-5'
3'-CCA CAT TTC CAG CAT CCC T-5'
3'-CCA CAT TTC CAG CAG CCC T-5'
3'-CCA CAT TTC CAG CAC CCC T-5'
ii. une séquence complémentaire d'une séquence telle que définie en i.; ou
iii. une séquence ayant au moins 60% d'identité avec l'une des séquences définies en i ou ii et capable d'hybrider à une température de 60°C±1°C avec les génomes des virus HIV-1 Bru, HIV-1 Mal, HIV-1 Eli, HIV-2 ROD et SIV Mac.

Un autre procédé selon l'invention est caractérisé en ce qu'au moins deux des mélanges d'amorces suivants sont utilisés pour l'amplification :

MMy1: Mélange constitué des amorces
5'-TGG CGC CCG AAC AGG GAC-3'
5'-TGG CGC CTG AAC AGG GAC-3'

MMy2 : Mélange constitué des amorces

5'-GGC CAG GGG GAA AGA AAA A-3'
5'-GGC CCG GCG GAA AGA AAA A-3'
5'-GGC CAG GAG GAA AGA AAA A-3'

MMy3 : Mélange constitué des amorces
3'-TGC CCA TAC AAA ATG TTT TA-5''
3'-TGC CCA CAC TAT ATG TTT TA-5'

MMy4 : Mélange constitué des amorces
3'-TGC ATG GCT GCT TGA TG-5'
3'-TGC ATA GCT GCC TGG TG-5'

MMy4B: Mélange constitué des amorces
3'-CTT TGC ATG GCT GCT TGA TG-5'
3'-CTC TGC ATA GCT GCC TGA TG-5'

MMy4Bbis: Mélange constitué des amorces
5′-CAT CAA GCA GCC ATG CAA AG-3′
5′-CAC CAG GCA GCT ATG CAG AG-3′

MMy28 : Mélange constitué des amorces
5'-AGG GCT GTT GGA AAT GTG G-3'
5'-AGG GCT GTT GGA AGT GTG G-3'

MMy28bis : Mélange constitué des amorces

3′-CCA CAT TTC CAG CAT CCC T-5′
3′-CCA CAT TTC CAG CAG CCC T-5′
3′-CCA CAT TTC CAG CAC CCC T-5′

Un autre procédé selon l'invention est caractérisé en ce qu'il est réalisé dans les conditions suivantes :
- pour l'étape d'hybridation : 1µl d'une solution à 40 µmolaire de chaque amorce est mis en présence des 100 à 300 ng d'ADN-matrice pour la première étape de dénaturation-réassociation ; on chauffe, durant 10 minutes à 100°C puis on plonge les tubes contenant ce mélange d'ADN-matrice et d'amorces dans de l'eau contenant de la glace, les amorces étant utilisées à une concentration finale dans l'étape d'amplification qui suit de 0,8 µM chaque ;
- pour l'étape d'amplification, on ajoute au milieu précédent les 4 dNTPs, chacun étant utilisé à 0,5 µmolaire dans 50 µl de solution finale, et une unité de Taq-polymérase pour un milieu réactionnel de 50 µl, cette étape étant réalisée dans le tampon d'amplification désigné sous le nom de « 10 X buffer » comprenant lorsqu'il est dilué au 1/10 dans la solution finale : Tris-HCl, pH = 8,9 : 50mM; (NH₄)₂ SO₄ ; 15 mM ; MgCl₂ ; 5 mM ; β-mercapto-éthanol : 10 mM ; gélatine : 0,25 mg/ml.

Un autre procédé selon l'invention est caractérisé en ce que l'étape de traduction est réalisée par transformation de cellules hôtes appropriées à l'aide de vecteurs contenant lesdites séquences amplifiées et récupération des protéines produites dans ces cellules hôtes.

Un autre procédé selon l'invention est caractérisé en ce que les amorces oligonucléotidiques sont choisies parmi les couples de mélanges d'amorces suivants :
a. MMy1-MMy4 
MMy1: Mélange constitué des amorces
5'-TGG CGC CCG AAC AGG GAC-3'
5'-TGG CGC CTG AAC AGG GAC-3'
 
MMy4 :Mélange constitué des amorces
3'-TGC ATG GCT GCT TGA TG-5'
3'-TGC ATA GCT GCC TGG TG-5'
b. MMy2-MMy4 
MMy2 :Mélange constitué des amorces
5'-GGC CAG GGG GAA AGA AAA A-3'
5'-GGC CCG GCG GAA AGA AAA A-3'
5'-GGC CAG GAG GAA AGA AAA A-3'
 
MMy4 :Mélange constitué des amorces
3'-TGC ATG GCT GCT TGA TG-5'
3'-TGC ATA GCT GCC TGG TG-5'
c. MMy1-MMy3 
MMy1: Mélange constitué des amorces
5'-TGG CGC CCG AAC AGG GAC-3'
5'-TGG CGC CTG AAC AGG GAC-3'
 
MMy3 : Mélange constitué des amorces
3'-TGC CCA TAC AAA ATG TTT TA-5''
3'-TGC CCA CAC TAT ATG TTT TA-5'
d MMy4Bbis-MMy28bis

MMy4Bbis: Mélange constitué des amorces
5'-CAT CAA GCA GCC ATG CAA AG-3'
5'-CAC CAG GCA GCT ATG CAG AG-3'

MMy28bis : Mélange constitué des amorces
3'-CCA CAT TTC CAG CAT CCC T-5'
3'-CCA CAT TTC CAG CAG CCC T-5'
3'-CCA CAT TTC CAG CAC CCC T-5'

La dernière étape de traduction est réalisée notamment par transformation de cellules hôtes appropriées à l'aide de vecteurs contenant lesdites séquences amplifiées, et récupération des protéines produites dans ces cellules hôtes.

Sont concernés également les polypeptides issus de la traduction des séquences (ou amorces) nucléotidiques de l'invention.

La présente demande décrit également les compositions immunogènes comprenant un ou plusieurs produits de traduction des séquences nucléotidiques selon l'invention, et/ou un ou plusieurs produits de traduction des séquences nucléotidiques amplifiées selon les procédés décrits ci-dessus à partir des amorces définies selon l'invention, ces produits de traduction étant associés à un véhicule pharmaceutiquement acceptable.

La présente demande décrit les anticorps dirigés contre l'un ou plusieurs des produits de traduction décrits ci-dessus (ou en d'autres termes, susceptibles de former une réaction immunologique avec un ou plusieurs produits de traduction des séquences nucléotidiques selon l'invention, ou encore un ou plusieurs produits de traduction des séquences nucléotidiques amplifiées à partir des amorces définies selon l'invention).

Un procédé de préparation des séquences (ou amorces) nucléotidiques décrites ci-dessus comprend les étapes suivantes :
- incubation de l'ADN génomique, isolé à partir d'un des virus du type HIV ou SIV süs-mentionnés, avec de l'ADNase I, puis addition d'EDTA et purification par extraction au mélange phenol/chloroforme/alcool isoamylique (25/24/ 1) puis par l'éther,
- traitement de l'ADN ainsi extrait par de l'Eco R1 méthylase en présence de DTT, et purification par extraction telle que décrite ci-dessus,
- incubation de l'ADN ainsi purifié avec les 4 désoxynucléotides triphosphates dATP, dCTP, dGTP, et dTTP en présence de T4 ADN polymérase et d'ADN ligase de E. coli, puis purification selon la méthode décrite ci-dessus,
- le clonage des acides nucléiques ainsi obtenus dans un vecteur approprié et la récupération de l'acide nucléique recherché à l'aide d'une sonde appropriée.

Un procédé de préparation particulièrement avantageux des séquences nucléotidiques de l'invention comprend les étapes suivantes :
- la synthèse d'ADN en utilisant la méthode automatisée des β-cyanethyl phosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un autre procédé de préparation des séquences nucléotidiques de l'invention comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

## Revendications

1. Procédé de synthèse d'une protéine ou d'un polypeptide codé par une séquence nucléotidique du virus HIV-1, HIV-2 ou SIV **caractérisé en ce qu'**il comprend les étapes suivantes :
a. synthèse de la séquence nucléotidique par un procédé d'amplification génique de séquences nucléotidiques de virus de type HIV-1, HIV-2 ou SIV, à l'aide d'au moins deux amorces oligonucléotidiques dont les séquences consistent chacune en
i. une séquence spécifique du gène gag choisie dans une région conservée entre les génomes des virus HIV-1 Bru, HIV-1 Mal, HIV-1 Eli, HIV-2 Rod et SIV Mac; capable d'hybrider à une température de 60°C ± 1°C avec les génomes des virus HIV-1 Bru, HIV-1 Mal, HIV-1 Eli, HIV-2 Rod et SIV Mac ou,
ii. une séquence complémentaire d'une séquence telle que définie en i., et
b. récupération de la séquence nucléotidique ainsi amplifiée et traduction en protéine.

2. Procédé de synthèse selon la revendication 1 dans lequel la séquence des amorces a au moins 60% d'identité avec la séquence du gène gag d'un virus HIV-1 Bru, ou HIV-1 Mal, ou HIV-1 Eli, ou HIV-2 ROD ou SIV Mac.

3. Procédé de synthèse selon la revendication 1, **caractérisé en ce que** les amorces oligonucléotidiques comprennent une conservation d'au moins 5 bases de chaque côté de l'amorce par rapport à la séquence du gène gag d'un virus HIV-1 Bru, HIV-1 Mal, HIV-1 Eli, ou HIV-2 ROD ou SIV Mac.; et comportent dans leur partie médiane des modifications et gardent les propriétés d'hybridation avec les génomes des virus HIV-1 Bru, HIV-1 Mal HIV-1 Eli, HIV-2 Rod et SIV Mac.

4. Procédé selon l'une des revendications 1 à 3, **caractérisé en ce que** le procédé d'amplification génique comprend les étapes suivantes :
a. une étape d'extraction de l'acide nucléique appartenant au génome du virus de type HIV-1, HIV-2 ou SIV, et éventuellement une étape de traitement à l'aide d'une transcriptase reverse dudit acide nucléique si ce dernier est sous forme d'ARN,
b. un cycle comprenant les étapes suivantes :
i. dénaturation de l'acide nucléique double brin à détecter, ce qui conduit à la formation d'un acide nucléique simple brin,
ii. hybridation de chacun des brins d'acide nucléique, obtenus lors de l'étape de dénaturation précédente, avec au moins une amorce nucléotidique, par mise en contact des brins sus-mentionnés avec au moins un couple d'amorces selon l'une des revendications 1 ou 2,
iii. formation, à partir des amorces, des ADN complémentaires aux brins sur lesquels lesdites amorces sont hybridées en présence d'une ADN polymérase et de quatre nucléosides triphosphates (dNTP) différents, ce qui conduit à la formation d'un plus grand nombre d'acides nucléiques double brin qu'à l'étape de dénaturation précédente,
ce cycle étant répété un nombre de fois déterminé pour obtenir ladite séquence nucléotidique dans une proportion suffisante pour permettre sa détection.

5. Procédé selon l'une quelconque des revendications 1 à 4, **caractérisé en ce que** les au moins deux amorces oligonucléotidiques ont des séquences consistant chacune en
i. au moins une séquence choisie dans le groupe des séquences sens suivant : 
5'-TGG CGC CCG AAC AGG GAC-3'
5'-TGG CGC CTG AAC AGG GAC-3'
5'-GGC CAG GGG GAA AGA AAA A-3'
5'-GGC CCG GCG GAA AGA AAA A-3'
5'-GGC CAG GAG GAA AGA AAA A-3'
5'-CAT CAA GCA GCC ATG CAA AG-3'
5'-CAC CAG GCA GCT ATG CAG AG-3'
5'-AGG GCT GTT GGA AAT GTG G-3'
5'-AGG GCT GTT GGA AGT GTG G-3'
 et au moins une séquence choisie dans le groupe des séquences antisens suivant 
3'-TGC CCA TAC AAA ATG TTT TA-5''
3'-TGC CCA CAC TAT ATG TTT TA-5'
3'-TGC ATG GCT GCT TGA TG-5'
3'-TGC ATA GCT GCC TGG TG-5'
3'-CTT TGC ATG GCT GCT TGA TG-5'
3'-CTC TGC ATA GCT GCC TGA TG-5'
3'-CCA CAT TTC CAG CAT CCC T-5'
3'-CCA CAT TTC CAG CAG CCC T-5'
3'-CCA CAT TTC CAG CAC CCC T-5'
ii. une séquence complémentaire d'une séquence telle que définie en i.; ou
iii. une séquence ayant au moins 60% d'identité avec l'une des séquences définies en i ou ii et capable d'hybrider à une température de 60°C±1°C avec les génomes des virus HIV-1 Bru, HIV-1 Mal, HIV-1 Eli, HIV-2 ROD et SIV Mac.

6. Procédé selon l'une quelconque des revendications 1 à 4, **caractérisé en ce qu'**au moins deux des mélanges d'amorces suivants sont utilisés pour l'amplification :

MMy1: Mélange constitué des amorces
5'-TGG CGC CCG AAC AGG GAC-3'
5'-TGG CGC CTG AAC AGG GAC-3'

MMy2 : Mélange constitué des amorces

5'-GGC CAG GGG GAA AGA AAA A-3'
5'-GGC CCG GCG GAA AGA AAA A-3'
5'-GGC CAG GAG GAA AGA AAA A-3'

MMy3 : Mélange constitué des amorces
3'-TGC CCA TAC AAA ATG TTT TA-5''
3'-TGC CCA CAC TAT ATG TTT TA-5'

MMy4 : Mélange constitué des amorces

3'-TGC ATG GCT GCT TGA TG-5'

3'-TGC ATA GCT GCC TGG TG-5'

MMy4B: Mélange constitué des amorces
3'-CTT TGC ATG GCT GCT TGA TG-5'
3'-CTC TGC ATA GCT GCC TGA TG-5'

MMy4Bbis: Mélange constitué des amorces
5'-CAT CAA GCA GCC ATG CAA AG-3'
5'-CAC CAG GCA GCT ATG CAG AG-3'

MMy28 : Mélange constitué des amorces
5'-AGG GCT GTT GGA AAT GTG G-3'
5'-AGG GCT GTT GGA AGT GTG G-3'

MMy28bis : Mélange constitué des amorces

3'-CCA CAT TTC CAG CAT CCC T-5'
3'-CCA CAT TTC CAG CAG CCC T-5'
3'-CCA CAT TTC CAG CAC CCC T-5'

7. Procédé selon la revendication 6, **caractérisé en ce qu'**il est réalisé dans les conditions suivantes :
- pour l'étape d'hybridation : 1 µl d'une solution à 40 µmolaire de chaque amorce est mis en présence des 100 à 300 ng d'ADN-matrice pour la première étape de dénaturation-réssociation ; on chauffe, durant 10 minutes à 100°C puis on plonge les tubes contenant ce mélange d'ADN-matrice et d'amorces dans de l'eau contenant de la glace, les amorces étant utilisées à une concentration finale dans l'étape d'amplification qui suit de 0,8 µM chaque;
- pour l'étape d'amplification, on ajoute au milieu précédent les 4 dNTPs, chacun étant utilisé à 0,5 µmolaire dans 50 µl de solution finale, et une unité de Taq-polymérase pour un milieu réactionnel de 50 µl, cette étape étant réalisée dans le tampon d'amplification désigné sous le nom de « 10 X buffer » comprenant lorsqu'il est dilué au 1/10 dans la solution finale : Tris-HCl, pH = 8,9 : 50mM; (NH₄)₂ SO₄ ; 15 mM ; MgCl₂ ; 5 mM ; β-mercapto-éthanol : 10 mM ; gélatine : 0,25 mg/ml.

8. Procédé selon l'une quelconque des revendications 1 à 7, **caractérisé en ce que** l'étape de traduction est réalisée par transformation de cellules hôtes appropriées à l'aide de vecteurs contenant lesdites séquences amplifiées et récupération des protéines produites dans ces cellules hôtes.

9. Procédé selon l'une quelconque des revendications 1 à 8, **caractérisé en ce que** les amorces oligonucléotidiques sont choisies parmi les couples de mélanges d'amorces suivants :
a. MMy1-MMy4 
MMy1: Mélange constitué des amorces
5'-TGG CGC CCG AAC AGG GAC-3'
5'-TGG CGC CTG AAC AGG GAC-3'
 
MMy4 :Mélange constitué des amorces
3′-TGC ATG GCT GCT TGA TG-5′
3′-TGC ATA GCT GCC TGG TG-5′
b. MMy2-MMy4 
MMy2 :Mélange constitué des amorces
5'-GGC CAG GGG GAA AGA AAA A-3'
5'-GGC CCG GCG GAA AGA AAA A-3'
5'-GGC CAG GAG GAA AGA AAA A-3'
 
MMy4 :Mélange constitué des amorces
3'-TGC ATG GCT GCT TGA TG-5'
3'-TGC ATA GCT GCC TGG TG-5'
c. MMy1-MMy3 
MMy1: Mélange constitué des amorces
5'-TGG CGC CCG AAC AGG GAC-3'
5'-TGG CGC CTG AAC AGG GAC-3'
 
MMy3 : Mélange constitué des amorces
3'-TGC CCA TAC AAA ATG TTT TA-5''
3'-TGC CCA CAC TAT ATG TTT TA-5'
d MMy4Bbis-MMy28bis

MMy4Bbis: Mélange constitué des amorces
5'-CAT CAA GCA GCC ATG CAA AG-3'
5'-CAC CAG GCA GCT ATG CAG AG-3'

MMy28bis : Mélange constitué des amorces
3'-CCA CAT TTC CAG CAT CCC T-5'
3'-CCA CAT TTC CAG CAG CCC T-5'
3'-CCA CAT TTC CAG CAC CCC T-5'

## Claims

1. A method for synthesizing a protein or a polypeptide encoded by a nucleotide sequence of the HIV-1, HIV-2 or SIV virus, **characterized in that** it comprises the following steps:
a. synthesizing the nucleotide sequence by a method for gene amplification of nucleotide sequences of the HIV-1, HIV-2 or SIV type virus, using at least two oligonucleotide primers the sequences of which each consist of:
i) a specific sequence of the gag gene selected from a conserved region in genomes of the HIV-1 Bru, HIV-1 Mal, HIV-1 Eli, HIV-2 Rod and SIV Mac viruses, capable of hybridizing at a temperature of 60°C ± 1°C with genomes of the HIV-1 Bru, HN-1 Mal, HIV-1 Eli, HIV-2 Rod and SIV Mac viruses; or
ii) a sequence complementary to a sequence as defined in i) above; and
b. recovering the amplified nucleotide sequence and translation into protein.

2. A synthesis method according to claim 1, in which the primer sequence has at least 60% identity with the sequence for the gag gene of a HIV-1 Bru or HIV-1 Mal or HIV-1 Eli or HIV-2 Rod virus or SIV Mac virus.

3. A synthesis method according to claim 1, **characterized in that** the oligonucleotide primers conserve at least 5 bases either side of the primer with respect to the sequence of the gag gene of a HIV-1 Bru, HIV-1 Mal, HIV-1 Eli, HIV-2 Rod or SIV Mac virus and comprise modifications in their median portion, and retain hybridization properties with genomes of the HIV-1 Bru, HIV-1 Mal, HIV-1 Eli, HIV-2 Rod and SIV Mac viruses.

4. A method according to one of claims I to 3, **characterized in that** the gene amplification method comprises the following steps:
a. a step for extracting the nucleic acid belonging to the genome of the HIV-1, HIV-2 or SIV type virus, and optionally, a treatment step using a reverse transcriptase for said nucleic acid if the latter is in the form of RNA;
b. a cycle comprising the following steps:
i) denaturing the double strand nucleic acid to be detected, resulting in the formation of a single strand nucleic acid;
ii) hybridizing each of the strands of nucleic acid obtained during the preceding denaturing step with at least one nucleotide primer, by bringing said strands into contact with at least one pair of primers in accordance with claim 1 or claim 2;
iii) forming, from the primers, complementary DNA to the strands on which said primers are hybridized in the presence of a DNA polymerase and four different nucleoside triphosphates (dNTP), resulting in the formation of a larger number of double strand nucleic acids than in the preceding denaturing step;
said cycle being repeated a predetermined number of times to obtain said nucleotide sequence in a proportion sufficient for its detection.

5. A method according to any one of claims 1 to 4, **characterized in that** said at least two oligonucleotide primers have sequences each consisting in:
i) at least one sequence selected from the following group of sense sequences: 
5'-TGG CGC CCG AAC AGG GAC-3'
5'-TGG CGC CTG AAC AGG GAC-3'
5'-GGC CAG GGG GAA AGA AAA A-3'
5'-GGC CCG GCG GAA AGA AAA A-3'
5'-GGC CAG GAG GAA AGA AAA A-3'
5'-CAT CAA GCA GCC ATG CAA AG-3'
5'-CAC CAG GCA GCT ATG CAG AG-3'
5'-AGG GCT GTT GGA AAT GTG G-3'
5'-AGG GCT GTT GGA AGT GTG G-3'
 and at least one sequence selected from the following group of antisense sequences: 
3'-TGC CCA TAC AAA ATG TTT TA-5''
3'-TGC CCA CAC TAT ATG TTT TA-5'
3'-TGC ATG GCT GCT TGA TG-5'
3'-TGC ATA GCT GCC TGG TG-5'
3'-CTT TGC ATG GCT GCT TGA TG-5'
3'-CTC TGC ATA GCT GCC TGA TG-5'
3'-CCA CAT TTC CAG CAT CCC T-5'
3'-CCA CAT TTC CAG CAG CCC T-5'
3'-CCA CAT TTC CAG CAC CCC T-5'
ii) a complementary sequence of a sequence as defined in i); or
iii) a sequence having at least 60% identity with one of the sequences defined in i) or ii) and capable of hybridizing at a temperature of 60°C±1°C with genomes of the HIV-1 Bru, HIV-1 Mal, HIV-1 Eli, HIV-2 Rod and SIV Mac viruses.

6. A method according to any one of claims 1 to 4, **characterized in that** at least two of the following mixtures of primers are used for amplification:

MMy1: mixture constituted by the following primers:

5'-TGG CGC CCG AAC AGG GAC-3'
5'-TGG CGC CTG AAC AGG GAC-3'

MMy2: mixture constituted by the following primers:

5'-GGC CAG GGG GAA AGA AAA A-3'
5'-GGC CCG GCG GAA AGA AAA A-3'
5'-GGC CAG GAG GAA AGA AAA A-3'

MMy3: mixture constituted by the following primers:
3'-TGC CCA TAC AAA ATG TTT TA-5''
3'-TGC CCA CAC TAT ATG TTT TA-5'

MMy4: mixture constituted by the following primers:
3'-TGC ATG GCT GCT TGA TG-5'
3'-TGC ATA GCT GCC TGG TG-5'

MMy4B: mixture constituted by the following primers:
3'-CTT TGC ATG GCT GCT TGA TG-5'
3'-CTC TGC ATA GCT GCC TGA TG-5'

MMy4Bbis: mixture constituted by the following primers:
5'-CAT CAA GCA GCC ATG CAA AG-3'
5'-CAC CAG GCA GCT ATG CAG AG-3'

MMy28: mixture constituted by the following primers:
5'-AGG GCT GTT GGA AAT GTG G-3'
5'-AGG GCT GTT GGA AGT GTG G-3'

MMy28bis: mixture constituted by the following primers:

3'-CCA CAT TTC CAG CAT CCC T-5'
3'-CCA CAT TTC CAG CAG CCC T-5'
3'-CCA CAT TTC CAG CAC CCC T-5'

7. A method according to claim 6, **characterized in that** it is carried out under the following conditions:
• for the hybridization step: bringing 1 µl of a 40 µmolar solution of each primer into the presence of 100 ng to 300 ng of DNA-matrix for the first denaturing-reassociation step; heating for 10 minutes to 100°C then plunging the tubes containing said mixture of DNA-matrix and primers into water containing ice, the primers being used, in the following amplification step, at a final concentration of 0.8 µM each;
• for the amplification step, adding the 4 dNTPs to the above medium, each being used at 0.5 µmolar in 50 µl of final solution, and one unit of Taq-polymerase for a reaction medium of 50 µl, said step being carried out in the amplification buffer designated "10X buffer" comprising, when diluted 1/10 in the final solution: Tris-HCl, pH = 8.9: 50 mM; (NH₄)₂SO₄: 15 mM; MgCl₂: 5 mM; β-mercaptoethanol: 10 mM; gelatin: 0.25 mg/ml.

8. A method according to any one of claims I to 7, **characterized in that** the translation step is carried out by transformation of suitable host cells using vectors containing said amplified sequences and recovering proteins produced in said host cells.

9. A method according to any one of claims 1 to 8, **characterized in that** the oligonucleotide primers are selected from the following pairs of mixtures of primers:
a. MMy1-MMy4 
MMy1: mixture constituted by the following primers:
5'-TGG CGC CCG AAC AGG GAC-3'
5'-TGG CGC CTG AAC AGG GAC-3'
 
MMy4: mixture constituted by the following primers:

3'-TGC ATG GCT GCT TGA TG-5'
3'-TGC ATA GCT GCC TGG TG-5'
b. MMy2-MMy4 
MMy2: mixture constituted by the following primers:
5'-GGC CAG GGG GAA AGA AAA A-3'
5'-GGC CCG GCG GAA AGA AAA A-3'
5'-GGC CAG GAG GAA AGA AAA A-3'
 
MMy4: mixture constituted by the following primers:

3'-TGC ATG GCT GCT TGA TG-5'
3'-TGC ATA GCT GCC TGG TG-5'
c. MMy1-MMy3 
MMy1: mixture constituted by the following primers:
5'-TGG CGC CCG AAC AGG GAC-3'
5'-TGG CGC CTG AAC AGG GAC-3'
 
MMy3: mixture constituted by the following primers:

3'-TGC CCA TAC AAA ATG TTT TA-5''
3'-TGC CCA CAC TAT ATG TTT TA-5'
d MMy4Bbis-MMy28bis

MMy4Bbis: mixture constituted by the following primers:
5'-CAT CAA GCA GCC ATG CAA AG-3'
5'-CAC CAG GCA GCT ATG CAG AG-3'

MMy28bis: mixture constituted by the following primers:

3'-CCA CAT TTC CAG CAT CCC T-5'
3'-CCA CAT TTC CAG CAG CCC T-5'
3'-CCA CAT TTC CAG CAC CCC T-5'

## Patentansprüche

1. Syntheseverfahren eines Proteins oder eines Polypeptids, welches durch eine Nukleotidsequenz des Virus HIV-1, HIV-2 oder SIV kodiert ist, **dadurch gekennzeichnet, dass** es die folgenden Schritte aufweist:
a. Synthese der Nukleotidsequenz durch ein Verfahren der Genamplifikation von Nukleotidsequenzen des Virus des Typs HIV-1, HIV-2 oder SIV mit Hilfe wenigstens zweier Starteroligonukleotide, deren Sequenzen bestehen aus:
i. einer spezifischen Sequenz des Gens gag, welche aus einer zwischen den Genomen des Virus HIV-1 Bru, HIV-1 Mal, HIV-1 Eli, HIV-2 Rod und SIV Mac erhaltenen Region ist und in der Lage ist, bei einer Temperatur von 60°C ±1°C an die Genomen der Viren HIV-1 Bru, HIV-1 Mal, HIV-1 Eli, HIV-2 Rod und SIV Mac zu hybridisieren; oder
ii.einer Komplementärsequenz einer wie unter i. definierten Sequenz; oder
b. Rückgewinnung der so amplifizierten Nukleotidsequenz und Übersetzung in Protein

2. Syntheseverfahren gemäß Anspruch 1, in welchem die Sequenz der Starter wenigstens 60% identisch ist zu der Sequenz des Gens gag eines Virus HIV-1 Bru oder HIV-1 Mal oder HIV-1 ELI oder HIV-2 ROD oder SIV-Mac.

3. Syntheseverfahren gemäß Anspruch 1, **dadurch gekennzeichnet, dass** die Oligonukleotidstarter einen Erhalt von wenigstens 5 Basen auf jeder Seite des Starters bezogen auf die Sequenz des Gens gag eines Virus HIV-1 Bru, HIV-1 Mal, HIV-1 Eli oder HIV-2-ROD oder SIV-Mac aufweisen und in ihrem mittleren Teil Modifikationen aufweisen und die Hybridisierungseigenschaften an die Genome des Virus HIV-1 Bru, HIV-1 Mal, HIV-1 Eli, HIV-2 Rod und SIV Mac bewahren.

4. Verfahren gemäß einem der Ansprüche 1 bis 3, **dadurch gekennzeichnet, dass** das Verfahren der Genamplifikation die folgenden Schritte aufweist:
a. einen Schritt der Extraktion von Nukleinsäure, welche zum Genom des Virus des Typs HIV-1, HIV-2 oder SIV gehört, und gegebenenfalls einen Schritt der Behandlung mittels einer reversen Transcriptase der Nukleinsäure, wenn diese letztgenannte in Form von RNA vorliegt,
b. einen Kreislauf, der die folgenden Schritte aufweist:
i. Denaturierung der zu detektierenden Doppelstrang-Nukleinsäure, was zur Bildung einer Einzelstrang-Nukleinsäure führt,
ii. Hybridisierung von jedem der Nukleinsäurestränge, die bei dem vorausgegangenen Schritt der Denaturierung erhalten wurden, an wenigstens einen Nukleotidstarter durch in Kontakt bringen der oben genannten Stränge mit wenigstens einem Starterpaar gemäß einem der Ansprüche 1 oder 2,
iii. ausgehend von den Startern Bilden von DNA, die zu den Strängen komplementär ist, auf welche die Starter in Gegenwart einer DNA-Polymerase und vier unterschiedlichen Nukleosidtriphosphaten (dNTP) hybridisiert sind, was zu der Bildung einer größeren Anzahl Doppelstrang-Nukleinsäuren als bei dem vorausgegangenen Schritt der Denaturierung führt,
wobei dieser Kreislauf eine bestimmte Anzahl von Malen wiederholt wird, um die Nukleotidsequenz in einer Menge zu erhalten, die ausreichend ist, um ihre Detektion zu ermöglichen,

5. Verfahren gemäß einem der Ansprüche 1 bis 4, **dadurch gekennzeichnet, dass** die wenigstens zwei Oligonukleotidstarter Sequenzen aufweisen, welche jeweils bestehen aus:
i. wenigstens einer Sequenz, die aus der folgenden Gruppe von Sense-Sequenzen gewählt ist: 
5'-TGG CGC CCG AAC AGG GAC-3'
5'-TGG CGC CTG AAC AGG GAC-3'
5'-GGC CAG GGG GAA AGA AAA A-3'
5'-GGC CCG GCG GAA AGA AAA A-3'
5'-GGC CAG GAG GAA AGA AAA A-3'
 
5'-CAT CAA GCA GCC ATG CAA AG-3'
5'-CAC CAG GCA GCT ATG CAG AG-3'
5'-AGG GCT GTT GGA AAT GTG G-3'
5'-AGG GCT GTT GGA AGT GTG G-3'
 und wenigstens einer Sequenz, die aus der folgenden Gruppe von Antisense-Sequenzen gewählt ist: 
3'-TGC CCA TAC AAA ATG TTT TA-5''
3'-TGC CCA CAC TAT ATG TTT TA-5'
3'-TGC ATG GCT GCT TGA TG-5'
3'-TGC ATA GCT GCC TGG TG-5'
3'-CTT TGC ATG GCT GCT TGA TG-5'
3'-CTC TGC ATA GCT GCC TGA TG-5'
3'-CCA CAT TTC CAG CAT CCC T-5'
3'-CCA CAT TTC CAG CAG CCC T-5'
3'-CCA CAT TTC CAG CAC CCC T-5'
ii. einer Komplementärsequenz einer wie unter i. definierten Sequenz; oder
iii. einer Sequenz, welche wenigstens 60% identisch ist zu einer der unter i. oder ii. definierten Sequenzen und welche in der Lage ist, bei einer Temperatur von 60°C ±1°C an die Genomen der Viren HIV-1 Bru, HIV-1 Mal, HIV-1 Eli, HIV-2 ROD und SIV Mac zu hybridisieren.

6. Verfahren gemäß einem der Ansprüche 1 bis 4, **dadurch gekennzeichnet, dass** wenigstens zwei der folgenden Startermischungen für die Amplifikation verwendet werden:

MMy1: Mischung, die gebildet ist aus den Startern
5'-TGG CGC CCG AAC AGG GAC-3'
5'-TGG CGC CTG AAC AGG GAC-3'

MMy2: Mischung, die gebildet ist aus den Startern
5'-GGC CAG GGG GAA AGA AAA A-3'
5'-GGC CCG GCG GAA AGA AAA A-3'
5'-GGC CAG GAG GAA AGA AAA A-3'

MMy3: Mischung, die gebildet ist aus den Startern
3'-TGC CCA TAC AAA ATG TTT TA-5''
3'-TGC CCA CAC TAT ATG TTT TA-5'

MMy4: Mischung, die gebildet ist aus den Startern
3'-TGC ATG GCT GCT TGA TG-5'
3'-TGC ATA GCT GCC TGG TG-5'

MMy4B: Mischung, die gebildet ist aus den Startern
3'-CTT TGC ATG GCT GCT TGA TG-5'
3'-CTC TGC ATA GCT GCC TGA TG-5'

MMy4Bbis: Mischung, die gebildet ist aus den Startern
5'-CAT CAA GCA GCC ATG CAA AG-3'
5'-CAC CAG GCA GCT ATG CAG AG-3'

MMy28: Mischung, die gebildet ist aus den Startern
5'-AGG GCT GTT GGA AAT GTG G-3'
5'-AGG GCT GTT GGA AGT GTG G-3'

MMy28bis: Mischung, die gebildet ist aus den Startern
3'-CCA CAT TTC CAG CAT CCC T-5'
3'-CCA CAT TTC CAG CAG CCC T-5'
3'-CCA CAT TTC CAG CAC CCC T-5'

7. Verfahren gemäß Anspruch 6, **dadurch gekennzeichnet, dass** es unter den folgenden Bedingungen durchgeführt wird:
- für den Schritt der Hybridisierung: 1 µl einer Lösung zu 40 µmol jedes Starters wird für den ersten Schritt der Denaturierung - Reassoziation mit 100 bis 300 ng DNA-Matrix in Gegenwart gebracht; es wird für 10 Minuten bei 100°C erhitzt, dann werden die Röhrchen, welche die Mischung aus DNA-Matrix und Startern enthalten, in Wasser getaucht, das Eis enthält, wobei die Starter mit einer End-Konzentration in dem Folgeschritt der Amplifikation von 0,8µM jeweils verwendet werden;
- für den Schritt der Amplifikation werden dem vorausgegangenen Medium die 4 dNTPs hinzugefügt, wobei jedes zu 0,5 µmol in 50 µl der Endlösung verwendet wird, und eine Gruppierung von Taq-Polymerase für ein Reaktionsmedium von 50 µl, wobei dieser Schritt in dem Amplifikationspuffer durchgeführt wird, der mit dem Namen "10 X buffer" bezeichnet wird, sofern dieser zu 1/10 in der Endlösung verdünnt ist: Tris-HCl, pH = 8,9 : 50mM; (NH₄)₂ SO₄: 15 mM; MgCl₂: 5mM; β-Mercaptoethanol: 10 mM; Gelatine: 0,25 mg/ml.

8. Verfahren gemäß einem der Ansprüche 1 bis 7, **dadurch gekennzeichnet, dass** der Schritt der Übersetzung durchgeführt wird durch Transformation von geeigneten Wirtszellen mittels Vektoren, welche die amplifizierten Sequenzen enthalten, und Rückgewinnung der in diesen Wirtszellen produzierten Proteine.

9. Verfahren gemäß einem der Ansprüche 1 bis 8, **dadurch gekennzeichnet, dass** die Oligonukleotidstarter aus den folgenden Paaren von Startermischungen gewählt sind:
a. MMy1-MMy4 
MMy1: Mischung, die gebildet ist aus den Startern
5'-TGG CGC CCG AAC AGG GAC-3'
5'-TGG CGC CTG AAC AGG GAC-3'
 
MMy4: Mischung, die gebildet ist aus den Startern
3'-TGC ATG GCT GCT TGA TG-5'
3'-TGC ATA GCT GCC TGG TG-5'
b. MMy2-MMy4 
MMy2: Mischung, die gebildet ist aus den Startern
5'-GGC CAG GGG GAA AGA AAA A-3'
5'-GGC CCG GCG GAA AGA AAA A-3'
5'-GGC CAG GAG GAA AGA AAA A-3'
 
MMy4: Mischung, die gebildet ist aus den Startern
3'-TGC ATG GCT GCT TGA TG-5'
3'-TGC ATA GCT GCC TGG TG-5'
c. MMy1-MMy3 
MMy1: Mischung, die gebildet ist aus den Startern
5'-TGG CGC CCG AAC AGG GAC-3'
5'-TGG CGC CTG AAC AGG GAC-3'
 
MMy3: Mischung, die gebildet ist aus den Startern
3'-TGC CCA TAC AAA ATG TTT TA-5''
3'-TGC CCA CAC TAT ATG TTT TA-5'
d. MMy4Bbis-MMy28bis
